# EUROPÄISCHE PATENTSCHRIFT

(11) **EP 0 745 582 B1**
(45) Veröffentlichungstag und Bekanntmachung des Hinweises auf die Patenterteilung: **23.08.2000**
(21) Anmeldenummer: 96108082.7
(22) Anmeldetag: 21.05.1996
(51) Int. Cl.: C07C 229/16, C07C 227/26, C07C 227/16, C07C 227/18

(54) **Verfahren zur Herstellung von Glycin-N,N-diessigsäure-Derivaten durch Umsetzung von Glycinderivaten oder deren Vorstufen mit Formaldehyd und Cyanwasserstoff oder von Iminodiacetonitril oder Iminodiessigsäure mit entsprechenden Aldehyden und Cyanwasserstoff in wässrig-saurem Medium**
Process for the preparation of glycine-N,N-diacetic acid derivatives from the reaction of glycine derivatives or their precursors with formaldehyde and hydrocyanic acid or of iminodiacetonitrile or iminodiacetic acid with appropriate aldehydes and hydrogen cyanide in an acidic aqueous medium
Procédé pour la préparation de dérivés de glycine-N,N-diacétique par réaction de dérivés de la glycine ou de leurs précurseurs avec le formaldéhyde et l'acide cyanhydrique ou d'iminodiacétonitrile ou d'acide iminodiacétique avec des aldéhydes correspondants et de l'acide cyanhydrique en milieu acide aqueux

(30) Priorität: 29.05.1995 DE 19518986
(43) Veröffentlichungstag der Anmeldung: 04.12.1996
(73) Patentinhaber: BASF Aktiengesellschaft, 67063 Ludwigshafen (DE)
(72) Erfinder: Greindl, Thomas, Dr., 94127 Neuburg (DE); Oftring, Alfred, Dr., 67098 Bad Dürkheim (DE); Braun, Gerold, Dr., 67071 Ludwigshafen (DE); Wild, Jochen, Dr., 67152 Ruppertsberg (DE); Potthoff-Karl, Birgit, Dr., 67061 Ludwigshafen (DE); Schuh, Georg, 67067 Ludwigshafen (DE)

(56) Entgegenhaltungen:
- EP-A- 0 287 885
- DE-A- 4 319 935

## Beschreibung

Die vorliegende Erfindung betrifft ein verbessertes Verfahren zur Herstellung von Glycin-N,N-diessigsäure-Derivaten durch Umsetzung von Glycinderivaten oder deren Vorstufen mit Formaldehyd und Cyanwasserstoff oder von Iminodiacetonitril oder Iminodiessigsäure mit entsprechenden Aldehyden und Cyanwasserstoff in wäßrigsaurem Medium.

In den typischen Anwendungsgebieten von Komplexbildnern und Buildern, wie hochalkalische Reiniger und Haushaltswaschmittel, werden heute als Standardprodukte Aminopolyphosphonate, Polycarboxylate oder Ethylendiamintetraessigsäure (EDTA) eingesetzt. Diese Produkte sind nur schwer biologisch abbaubar, deshalb besteht ein Bedarf nach wirksamen und gleichzeitig billigen biologisch leicht abbaubaren Substituten.

Eine Alternative zu obigen Stoffen stellt Nitrilotriessigsäure (NTA) dar, sie ist leicht biologisch abbaubar, hat aber deutliche Wirkungsnachteile gegenüber EDTA und ist aus toxikologischen Gründen häufig unerwünscht. Methylglycin-N,N-diessigsäure (α-Alanin-N,N-diessigsäure, MGDA) ist ein nicht toxischer, leicht biologisch abbaubarer Komplexbildner mit höherer Komplexbildungskonstante als NTA. Die Verwendung von MGDA und verwandter Glycin-N,N-diessigsäure-Derivate für den Wasch- und Reinigungsmittelsektor und für zahlreiche neue Anwendungen sowie neue Syntheserouten für solche Substanzen werden in der WO-A 94/29421 (1) beschrieben.

Die Synthese von MGDA mit Hilfe von Chloressigsäure ist seit langem bekannt. Dieser Weg ist heute aufgrund des Zwangsanfalls von Natriumchlorid und der Bildung chlororganischer Verunreinigungen nicht mehr wirtschaftlich und vom ökologischen Standpunkt her auch nicht mehr zeitgemäß. Auch muß, um hohe Ausbeuten zu erreichen, Chloressigsäure im Überschuß eingesetzt werden, dabei kommt es zur Bildung von Glykolsäure, Oxodiacetat und chlororganischen Verbindungen als Nebenprodukten. Andere Halogenessigsäuren bilden ein ähnliches Nebenproduktspektrum aus. Die Abtrennung der stöchiometrisch anfallenden anorganischen Salze wie Natriumchlorid ist aufwendig und kostspielig.

Eine wirtschaftliche und gleichzeitig umweltschonende Methode zur Herstellung von Aminopolycarboxylaten ist prinzipiell die Strekker-Reaktion von Aminosäuren. Die Synthese von MGDA mit Hilfe der Strecker-Reaktion wird in (1) beschrieben.

In der DE-A 20 27 972 (2) wird die "saure" Variante der Strecker-Reaktion von unsubstituiertem Glycin mit Formaldehyd und Blausäure beschrieben. Hierbei bildet sich aus Glycin das N,N-Bis(cyanmethyl)glycin, welches in hoher Reinheit isoliert werden kann, nachteilhaft bei dem beschriebenen Verfahren ist der notwendige Einsatz von zusätzlicher Säure zur Erniedrigung des pH-Wertes sowie die Verwendung von teurem reinen Glycin. Das sich dabei bildende Glycin-N,N-diacetonitril wird zum Einsatz als Vernetzer beschrieben, die mögliche Verseifung zur Nitrilotriessigsäure ist nicht Gegenstand von (2). Die Umsetzung von Alanin mittels Strecker-Reaktion zu MGDA wird erstmals in (1) beschrieben, MGDA wird hier nach Verseifung in hohen Ausbeuten mit hoher Reinheit gewonnen, allerdings wird auch hier nur reines Alanin eingesetzt. Der hohe Preis für handelsübliche reine Aminosäuren wie Alanin stellt eine große wirtschaftliche Hürde für den Einsatz bei der Synthese von Glycin-N,N-diessigsäuren I dar.

Die "alkalische" Variante der Strecker-Reaktion wird beispielsweise in der US-A 3 733 355 (3) in allgemeiner Form dargestellt. Die dort aufgeführten Beispiele zeigen jedoch, daß immer ein hoher Anteil an Nebenprodukten, vor allem an unerwünschter Glykolsäure, auftritt; dies läßt sich aus den Umsätzen von nur maximal ca. 89 % schließen.

Ein Vorteil der "sauren" Variante gegenüber der "alkalischen" bei der Strecker-Reaktion ist die höhere Selektivität und die Möglichkeit, durch Isolierung anfallender Nitrilverbindungen einen zusätzlichen Reinigungsschritt einzuführen und so zu reineren Produkten zu gelangen.

In der US-A 2 500 019 (4) wird die Umsetzung von α-Aminosäuren mit Formaldehyd und Natriumcyanid allgemein erwähnt und am Beispiel von unsubstituiertem Proteinhydrolysat Nitrilotriessigsäure hergestellt. Bei diesem Verfahren wird allerdings mit Glycin eine besonders reaktive, weil unsubstituierte Aminosäure eingesetzt. Das sich bildende NTA ist zudem aufgrund seiner hohen Symmetrie thermodynamisch gegenüber unsymmetrischen Verbindungen bevorzugt und bildet sich besonders leicht.

Gerade die Umsetzung von stärker sterisch gehinderten Aminosäuren, wie etwa Alanin, in hohen Ausbeuten mit möglichst geringen Anteilen an NTA ist schwierig. In (4) wird Glycin-Natrium-Salz aus Proteinhydrolysat hergestellt. Ein Proteinhydrolysat enthält in der Regel Beimengungen von anderen Aminosäuren, so daß die Umsetzung nach Strecker hier zu keinem NTA-reinen Produkt führt.

Der Nachteil der Methode gemäß (4) liegt in der Bildung von Nebenprodukten, vor allem von Reaktionsprodukten des entstehenden Ammoniaks, und der geringen Stabilität der Einsatzstoffe bei den auftretenden hohen pH-Werten. Der Vorteil der einstufigen Fahrweise ergibt gleichzeitig den Nachteil, auftretende Zwischenverbindungen nicht isolieren und aufreinigen zu können.

Aufgabe der vorliegenden Erfindung war daher die Bereitstellung einer einfachen und wirtschaftlichen Syntheseroute für Glycin-N,N-diessigsäuren wie MGDA ausgehend von preiswerten Ausgangsmaterialien möglichst ohne zwischengeschaltete Reinigungsschritte, dabei sollte eine möglichst hohe Gesamtausbeute bei gleichzeitig hohen Produktreinheiten, mit niedrigen NTA-Gehalten, möglichst unter 2 Gew.-%, angestrebt werden.

Demgemäß wurde ein Verfahren zur Herstellung von Glycin-N,N-diessigsäure-Derivaten der allgemeinen Formel I 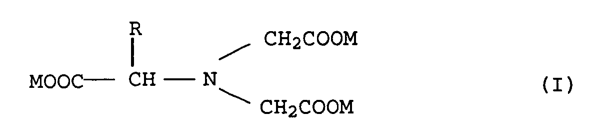 in der
- R: für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, Alkoxylat-Gruppierungen der Formel -(CH₂)ₖ-O-(A¹O)ₘ-(A²O)ₙ-Y, in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, Phenyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen oder C₁- bis C₄-Alkoxycarbonylgruppen tragen können, oder einen Rest der Formel 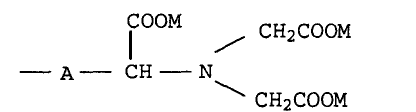 steht, wobei A eine C₁- bis C₁₂-Alkylen-Brücke oder eine chemische Bindung bezeichnet, und
- M: Wasserstoff, Alkalimetall, Erdalkalimetall, Ammonium oder substituiertes Ammonium in den entsprechenden stöchiometrischen Mengen bedeutet,
durch Umsetzung von
A) entsprechenden 2-substituierten Glycinen oder 2-substituierten Glycinnitrilen oder verdoppelten Glycinen der Formel 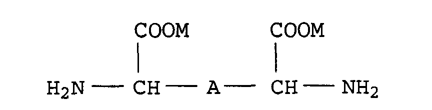 oder verdoppelten Glycinntrilen der Formel 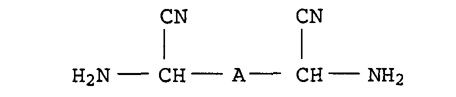 oder von Alaninaminonitril oder 5-Methylhydantoin als Vorstufen der als Ausgangsmaterial genannten Glycinderivate mit Formaldehyd und Cyanwasserstoff in wäßrigem Medium bei einem pH-Wert von 0 bis 8 oder
B)a) Iminodiacetonitril mit entsprechenden Monoaldehyden der Formel R-CHO oder Dialdehyden der Formel OHC-A-CHO und Cyanwasserstoff in wäßrigem Medium bei einem pH-Wert von 0 bis 5 oder
B)b) Iminodiessigsäure mit entsprechenden Monoaldehyden der Formel R-CHO oder Dialdehyden der Formel OHC-A-CHO und Cyanwasserstoff in wäßrigem Medium bei einem Ausgangs-pH-Wert von 1 bis 8
und gegebenenfalls anschließende Verseifung vorliegender Nitrilfunktionen gefunden, welches dadurch gekennzeichnet ist, daß man als Ausgangsmaterial aus der technischen Synthese von Glycinderivaten oder deren Vorstufen oder von Iminodiacetonitril oder Iminodiessigsäure stammendes nicht gereinigtes, d.h. in der Regel nicht als Feststoff isoliertes oder z.B. durch Kristallisation von Nebenbestandteilen befreites Rohmaterial oder bei solchen Synthesen anfallende Mutterlaugen einsetzt.

Unter Vorstufen von Glycinderivaten sind im Falle von Alanin (R=CH₃) Alaninaminonitril oder 5-Methylhydantoin zu verstehen, wobei letzteres z.B. durch Umsetzung von Acetaldehyd, Alkalimetallcyanid und Ammoniumcarbonat entsteht. Die übliche technische Alanin-Synthese wird nach Strecker durch Umsetzung von Acetaldehyd, Blausäure und Ammoniak durchgeführt. Auch enzymatisch hergestelltes Alanin kann ohne Feststoffisolierung eingesetzt werden. Nicht störende Nebenkomponenten in Alanin-Mutterlaugen können z.B. Milchsäure, Alaninnitril, 5-Methylhydantoin, Natriumsulfat, Ammoniumsulfat, Natriumcarbonat, Phosphatpuffer und andere Neutralpuffer sein.

Die erfindungsgemäße Umsetzung der eingesetzten Glycinderivate oder deren Vorstufen mit Formaldehyd und Cyanwasserstoff gemäß Ausführungsform A wird üblicherweise bei Temperaturen von 0 bis 100°C, insbesondere 10 bis 80°C, vor allem 20 bis 60°C durchgeführt. Der pH-Wert des wäßrigen Umsetzungsmediums liegt bei 0 bis 8, also im sauren oder auch im schwach alkalischen Bereich.

Man setzt bei Ausführungsform A pro Mol als Ausgangsmaterial verwendetem Glycinderivat oder dessen Vorstufe zweckmäßigerweise 2,0 bis 3,0, insbesondere 2,0 bis 2,6 mol Formaldehyd, vorzugsweise in Form seiner wäßrigen ca. 30 gew.-%igen Lösung, und 2,0 bis 3,0 mol, insbesondere 2,0 bis 2,6 mol Cyanwasserstoff ein. Üblicherweise verwendet man als Ausgangsmaterial wäßrige Lösungen der entsprechenden Glycinderivate bzw. Vorstufen mit einem Glycinderivat- bzw. Vorstufen-Gehalt von 10 bis 50 Gew.-%, insbesondere 25 bis 45 Gew.-%.

Normalerweise führt man die Umsetzung gemäß Ausführungsform A so durch, daß man die Glycinderivate oder deren Vorstufen vorlegt und bei der genannten Umsetzungstemperatur und dem genannten pH-Wert den Formaldehyd und den Cyanwasserstoff gleichzeitig über einen Zeitraum von 0,1 bis 12 Stunden, insbesondere 0,15 bis 6 Stunden, vor allem 0,25 bis 3 Stunden zudosiert. Anschließend läßt man üblicherweise noch 1 bis 20 Stunden, vorzugsweise 2 bis 5 Stunden unter den Umsetzungsbedingungen nachreagieren.

Der Formaldehyd wird normalerweise als wäßrige Lösung eingesetzt. Man kann aber auch diese Komponente beispielsweise in fester Form (z.B. als Paraformaldehyd) zugeben. Als Umsetzungsmedium dient bei Ausführungsform A und B in der Regel Wasser, das die Endprodukte sowie die eingesetzten Umsetzungskomponenten meist in ausreichendem Maße löst. Man kann aber auch Mischungen aus Wasser und wassermischbaren organischen Lösungsmitteln wie Alkoholen, z.B. Methanol, Ethanol oder Isopropanol, verwenden, wenn beispielsweise Glycin-N,N-diessigsäuren I mit einem hydrophoberen, d.h. längerkettigen oder voluminöseren Rest R hergestellt werden sollen.

Die Verseifung (Hydrolyse) von nach erfolgter Umsetzung vorliegender Nitrilfunktionen zu Carboxylatgruppen führt man üblicherweise mit 0,8 bis 2,0, insbesondere 1,0 bis 1,5 Moläquivalenten pro Nitrilfunktion wäßriger Natron- oder Kalilauge unter üblichen Bedingungen durch.

Auch Iminodiacetonitril- und Iminodiessigsäure-Rohgemische oder Mutterlaugen, welche beispielsweise bei der Alanin-Synthese nach Strecker anfallen können, können gemäß Ausführungsform B vorteilhaft eingesetzt werden. Nicht störende Nebenkomponenten in solchen Mutterlaugen können z.B. Glycinnitril, Alaninnitril, Milchsäurenitril, Glykolnitril, Methylen-bisiminodiacetonitril, Ammoniumsulfat sowie Nitrilotriacetonitril in geringen Mengen von < 0,5 Gew.-% sein.

Die erfindungsgemäße Umsetzung von Iminodiacetonitril oder Iminodiessigsäure mit den entsprechenden Aldehyden und Cyanwasserstoff gemäß Ausführungsform B erfolgt entweder durch Umsetzung von rohem Iminodiacetonitril oder Iminodiacetonitril enthaltenden Mutterlaugen mit dem Aldehyd und HCN zum entsprechenden Glycinnitril-N,N-diacetonitril und anschließende alkalische Hydrolyse zu den Verbindungen I (Weg a) oder durch alkalische Hydrolyse des Iminodiacetonitrils zur Iminodiessigsäure und anschließende Umsetzung mit dem Aldehyd und HCN zu den Verbindungen I (Weg b).

Iminodiacetonitril selbst ist beispielsweise nach bekannten Verfahren aus wäßrigem Urotropin durch Umsetzung mit Cyanwasserstoff (6 bis 8 Moläquivalente) in mineralsaurem Medium (pH 3 bis 8) bei 20 bis 100°C gut zugänglich. Man kann Iminodiacetonitril jedoch auch vorteilhaft direkt aus entsprechenden Mengen Formaldehyd, Ammoniak und HCN in beispielsweise wäßriger Schwefelsäure erzeugen.

Bei Weg (a) setzt man in der Regel das Iminodiacetonitril, vorzugsweise als 5 bis 30 gew.-%ige Mutterlauge, mit 0,8 bis 3,0 mol, insbesondere 1,0 bis 1,5 mol Cyanwasserstoff und gleichzeitig oder zeitversetzt mit 0,8 bis 3,0 mol, insbesondere 1,0 bis 1,5 mol des Aldehyds in wäßrigem Medium mit einem pH-Wert von 0 bis 5, welcher üblicherweise durch Zusatz von Mineralsäuren eingestellt wird, bei Temperaturen von 0 bis 100°C, insbesondere 20 bis 60°C, innerhalb von 0,5 bis 12 Stunden, insbesondere 1 bis 5 Stunden, um und läßt üblicherweise noch 0,5 bis 20 Stunden, insbesondere 2 bis 6 Stunden, bei den Umsetzungsbedingungen nachreagieren. Anschließend wird - gegebenenfalls nach Isolierung des Zwischenproduktes durch Filtrieren oder Abdekantieren - normalerweise mit 2 bis 5 Moläquivalenten, insbesondere 3 bis 4 Moläquivalenten wäßriger Natron- oder Kalilauge, bezogen auf eingesetztes Iminodiacetonitril, unter üblichen Bedingungen verseift.

Bei Weg (b) verseift man üblicherweise das erhaltene Iminodiacetonitril, gegebenenfalls nach Abtrennung durch Filtrieren oder Abdekantieren, mit 1,8 bis 3,0 Moläquivalenten, insbesondere 2,0 bis 2,5 Moläquivalenten wäßriger Natron- oder Kalilauge unter üblichen Bedingungen und säuert anschließend durch Zugabe von Mineralsäuren auf pH 1 bis 8 an. Danach wird die erhaltene Iminodiessigsäure in der Regel mit 0,8 bis 2,0 mol, insbesondere 1,0 bis 1,5 mol Cyanwasserstoff und gleichzeitig oder zeitversetzt mit 0,8 bis 2,0 mol, insbesondere 1,0 bis 1,5 mol des Aldehyds, jeweils bezogen auf eingesetztes Iminodiacetonitril, bei Temperaturen von 0 bis 100°C, insbesondere 20 bis 60°C, innerhalb von 1 bis 20 Stunden, insbesondere 2 bis 6 Stunden umgesetzt, anschließend läßt man üblicherweise noch 1 bis 20 Stunden, insbesondere 2 bis 10 Stunden, bei den Umsetzungsbedingungen nachreagieren. Abschließend wird normalerweise mit 0,8 bis 2,0 Moläquivalenten, insbesondere 1,0 bis 1,5 Moläquivalenten wäßriger Natron- oder Kalilauge, bezogen auf eingesetztes Iminodiacetonitril, unter üblichen Bedingungen zu den Verbindungen I verseift.

Es hat sich bei Verseifungsschritten des erfindungsgemäßen Verfahrens gemäß Ausführungsform A oder B als zweckmäßig erwiesen, vor und/oder während der Umsetzung ein Vakuum von 10⁴ bis 10⁵ Pa (100 bis 1000 mbar), vorzugsweise 3 x 10⁴ bis 9 x 10⁴ Pa (300 bis 900 mbar), insbesondere 5 x 10⁴ bis 8 x 10⁴ Pa (500 bis 800 mbar), an die Reaktionsapparatur anzulegen. Weiterhin kann man zusätzlich hierzu oder auch als Einzelmaßnahme vor und/oder während eines Verseifungsschrittes ein Inertgas wie Luft, Stickstoff oder Argon durch die Reaktionsmischung bzw. die vorgelegten Umsetzungskomponenten leiten ("Strippung" mit Inertgas). Das Vakuum und das Durchleiten von Inertgas dienen insbesondere dazu, noch aus den Vorstufen enthaltenen bzw. während der Verseifung sich bildenden Ammoniak aus dem Reaktionssystem besser zu entfernen.

Mit besonders guten Ergebnissen kann das erfindungsgemäße Verfahren zur Herstellung von Glycin-N,N-diessigsäure-Derivaten I angewandt werden, bei denen R für C₁- bis C₂₀-Alkyl, C₂- bis C₂₀-Alkenyl oder einen Rest der Formel 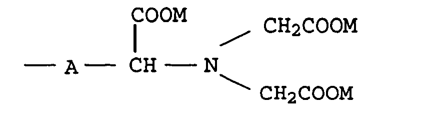 steht.

Ganz besonders gut eignet sich das erfindungsgemäße Verfahren zur Herstellung von α-Alanin-N,N-diessigsäure (R=CH₃) und ihren Alkalimetall-, Ammonium- und substituierten Ammoniumsalzen.

Als derartige Salze kommen vor allem die Natrium-, Kalium- und Ammoniumsalze, insbesondere das Trinatrium-, Trikalium- und Triammoniumsalz, sowie organische Triaminsalze mit einem tertiären Stickstoffatom in Frage.

Als den organischen Aminsalzen zugrundeliegenden Basen kommen insbesondere tertiäre Amine wie Trialkylamine mit 1 bis 4 C-Atomen im Alkyl, z.B. Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als Erdalkalimetallsalze werden insbesondere die Calcium- und Magnesiumsalze eingesetzt.

Neben Methyl kommen für den Rest R als geradkettige oder verzweigte Alk(en)ylreste vor allem C₂- bis C₁₇-Alkyl und -Alkenyl, hierbei insbesondere geradkettige, von gesättigten oder ungesättigten Fettsäuren abgeleitete Reste, in Betracht. Beispiele für einzelne Reste R sind: Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, Neopentyl, n-Hexyl, n-Heptyl, 3-Heptyl (abgeleitet von 2-Ethylhexansäure), n-Octyl, iso-Octyl (abgeleitet von iso-Nonansäure), n-Nonyl, n-Decyl, n-Undecyl, n-Dodecyl, iso-Dodecyl (abgeleitet von iso-Tridecansäure), n-Tridecyl, n-Tetradecyl, n-Pentadecyl, n-Hexadecyl, n-Heptadecyl, n-Octadecyl, n-Nonadecyl, n-Eicosyl und n-Heptadecenyl (abgeleitet von Ölsäure). Es können für R auch Gemische auftreten, insbesondere solche, die sich von natürlich vorkommenden Fettsäuren und von synthetisch erzeugten technischen Säuren, beispielsweise durch Oxosynthese, ableiten.

Als Beispiele für die weiterhin genannten C₁- bis C₄-, C₁- bis C₁₂- und C₁- bis C₂₀-Alkylgruppen sind auch die entsprechenden oben aufgeführten Reste für R zu verstehen.

Als C₁- bis C₁₂-Alkylen-Brücken A dienen vor allem Polymethylengruppierungen der Formel -(CH₂)ₖ-, worin k eine Zahl von 2 bis 12, insbesondere von 2 bis 8 bezeichnet, d.h. 1,2-Ethylen, 1,3-Propylen, 1,4-Butylen, Pentamethylen, Hexamethylen, Heptamethylen, Octamethylen, Nonamethylen, Decamethylen, Undecamethylen und Dodecamethylen. Hexamethylen, Octamethylen, 1,2-Ethylen und 1,4-Butylen werden hierbei besonders bevorzugt. Daneben können aber auch verzweigte C₁- bis C₁₂-Alkylengruppen auftreten, z.B. -CH₂CH(CH₃)CH₂-, -CH₂C(CH₃)₂CH₂-, -CH₂CH(C₂H₅)- oder -CH₂CH(CH₃)-.

Die C₁- bis C₃₀-Alkyl- und C₂- bis C₃₀-Alkenylgruppen können bis zu 5, insbesondere bis zu 3 zusätzliche Substituenten der genannten Art tragen und durch bis zu 5, insbesondere bis zu 3 nicht benachbarte Sauerstoffatome unterbrochen sein. Beispiele für solche substituierten Alk(en)ylgruppen sind -CH₂OH, -CH₂CH₂OH, -CH₂CH₂-O-CH₃, -CH₂-CH₂-O-CH₂CH₂-O-CH₃, -CH₂-O-CH₂CH₃, -CH₂-O-CH₂CH₂-OH, -CH₂-CHO, -CH₂-OPh, -CH₂-COOCH₃ oder CH₂CH₂-COOCH₃.

Als Alkoxylat-Gruppierungen kommen insbesondere solche in Betracht, bei denen m und n jeweils für Zahlen von 0 bis 30, vor allem von 0 bis 15 stehen. A¹ und A² bedeuten von Butylenoxid und vor allem von Propylenoxid und von Ethylenoxid abgeleitete Gruppen. Von besonderem Interesse sind reine Ethoxylate und reine Propoxylate, aber auch Ethylenoxid-Propylenoxid-Blockstrukturen können auftreten.

Als fünf- oder sechsgliedrige ungesättigte oder gesättigte heterocyclische Ringe mit bis zu drei Heteroatomen aus der Gruppe Stickstoff, Sauerstoff und Schwefel, welche zusätzlich benzanelliert und durch die bezeichneten Reste substituiert sein können, kommen in Betracht:

Tetrahydrofuran, Furan, Tetrahydrothiophen, Thiophen, 2,5-Dimethylthiophen, Pyrrolidin, Pyrrolin, Pyrrol, Isoxazol, Oxazol, Thiazol, Pyrazol, Imidazolin, Imidazol, 1,2,3-Triazolidin, 1,2,3- und 1,2,4-Triazol, 1,2,3-, 1,2,4- und 1,2,5-Oxadiazol, Tetrahydropyran, Dihydropyran, 2H- und 4H-Pyran, Piperidin, 1,3- und 1,4-Dioxan, Morpholin, Pyrazan, Pyridin, α-, β- und γ-Picolin, α- und γ-Piperidon, Pyrimidin, Pyridazin, Pyrazin, 1,2,5-Oxathiazin, 1,3,5-, 1,2,3- und 1,2,4-Triazin, Benzofuran, Thionaphthen, Indolin, Isoindolin, Benzoxazol, Indazol, Benzimidazol, Chroman, Isochroman, 2H- und 4H-Chromen, Chinolin, Isochinolin, 1,2,3,4-Tetrahydroisochinolin, Cinnolin, Chinazolin, Chinoxalin, Phthalazin und Benzo-1,2,3-triazin.

N-H-Gruppierungen in den genannten heterocyclischen Ringen sollten möglichst in derivatisierter Form, etwa als N-Alkyl-Gruppierung, vorliegen.

Bei Substitution an den Phenylkernen oder den heterocyclischen Ringen treten vorzugsweise zwei (gleiche oder verschiedene) oder insbesondere ein einzelner Substituent auf.

Beispiele für gegebenenfalls substituierte Phenylalkylgruppen und heterocyclische Ringe tragende Alkylgruppen für R sind Benzyl, 2-Phenylethyl, 3-Phenylpropyl, 4-Phenylbutyl, o-, m- oder p-Hydroxylbenzyl, o-, m- oder p-Carboxylbenzyl, o-, m- oder p-Sulfobenzyl, o-, m- oder p-Methoxy- oder -Ethoxycarbonylbenzyl, 2-Furylmethyl, N-Methylpiperidin-4-ylmethyl oder 2-, 3- oder 4-Pyridinylmethyl.

Bei Substitution an Phenylkernen und auch an heterocyclischen Ringen treten bevorzugt wasserlöslich machende Gruppen wie Hydroxylgruppen, Carboxylgruppen oder Sulfogruppen auf.

Die nach dem erfindungsgemäßen Verfahren hergestellten Verbindungen I können hinsichtlich des α-C-Atoms als Racemate oder als enantiomerenreine Verbindungen vorliegen, je nachdem, ob man bei Ausführungsform A von D,L-Glycinderivaten oder den entsprechenden D- oder L-Formen ausgeht. Bei Ausführungsform B entstehen in der Regel die Racemate von I.

Die freien Säuren der Verbindungen I können, falls die Verbindungen I als Salze anfallen, durch Ansäuern nach üblichen Methoden erhalten werden.

Beim erfindungsgemäßen Verfahren wird durch die spezielle Reaktionsführung die Bildung von unerwünschtem NTA im Produkt weitestgehend unterdrückt, die NTA-Mengen liegen deutlich unter 2 Gew.-%, meist bei 0,1 bis 0,3 Gew.-%. Bei Ausführungsform B wird analog die Bildung von Nitrilotriacetonitril unterdrückt.

Gerade durch den Einsatz von Mutterlaugen beim erfindungsgemäßen Verfahren kann der Aufarbeitungsverlust bei der Isolierung der Glycinderivate oder deren Vorstufen bzw. bei der Isolierung von Iminodiacetonitril vermieden werden. Ebenso kann eine sonst als separater Zwischenschritt anfallende Verseifung einer Nitril-Zwischenstufe vermieden werden. Somit ist das erfindungsgemäße Verfahren wesentlich wirtschaftlicher. Die Effektivität der Umsetzung der Ausgangsverbindungen in Mutterlaugen wird überraschenderweise im Vergleich zur Umsetzung der reinen Ausgangsverbindungen nicht beeinträchtigt, so daß die Erhöhung der Gesamtausbeute meist 5 bis 15 % beträgt.

Der zentrale Aspekt des erfindungsgemäßen Verfahrens ist die Möglichkeit, anstelle von reinen Glycinderivate bzw. reinem Iminodiacetonitril oder reiner Iminodiessigsäure Rohgemische, wie sie z.B. bei der Aminosäure-Synthese nach Strecker, etwa von Alanin, oder auch enzymatisch entstehen, aber auch entsprechende Vorstufen wie 5-Methylhydantoine einzusetzen. Diese Vorgehensweise ist besonders wirtschaftlich, da die im Anschluß an die Aminosäureherstellung normalerweise notwendige teure Produktabtrennung am isoelektrischen Punkt hier nicht notwendig ist. Somit können Reagenzien zur pH-Wert-Einstellung eingespart und Abtrennungsverluste vermieden werden, da auch die normalerweise in der Mutterlauge der Aminosäuresynthese verbleibende Aminosäure genutzt wird. Günstigerweise kann das Alkalimetallsalz aus der Aminosäuresynthese ohne weiteren Zusatz von Alkali direkt und ohne Ausbeute- und Selektivitätsverlust umgesetzt werden. 5-Methylhydantoin kann ebenfalls wie Nitrile in einem Ansatz durch Zugabe von entsprechender Menge Alkali verseift und unmittelbar in einem Arbeitsgang mit Formaldehyd und Cyanwasserstoff in erfindungsgemäßer Weise umgesetzt werden, insgesamt liefert diese Methode gegenüber der Umsetzung von durch Fällung isolierter Aminosäure eine höhere Gesamtausbeute bei gleichzeitig einfacherem Verfahren.

Die besondere Verfahrensweise des erfindungsgemäßen Verfahrens erlaubt es auch, die Synthese nicht nur diskontinuierlich, sondern auch kontinuierlich, ausgehend von billig verfügbaren chemischen Grundstoffen wie Formaldehyd, Acetaldehyd, Blausäure, Ammoniak und Natronlauge, in einer Folge von aneinander gereihten Reaktionsstufen durchzuführen, wobei Ausbeute und Reinheit bei gleichzeitig besonders hoher Wirtschaftlichkeit nicht beeinträchtigt werden. Insbesondere die Umsetzung von Iminodiacetonitril zum entsprechenden Glycinnitril-N,N-diacetonitril (Weg a) und die anschließende Verseifung zu I eignen sich für ein solches kontinuierliches Verfahren. Durch die kontinuierliche Fahrweise wird überraschenderweise vor allem bei der Verseifung der NTA-Gehalt im Endprodukt noch weiter reduziert.

### Beispiele

### Beispiel 1

### Herstellung von MGDA-Trinatriumsalz aus Alanin-Rohgemisch

Zu einer Suspension von 44 g D,L-Alanin, hergestellt durch die Umsetzung von 27,5 g Acetaldehyd mit 17 g Blausäure (99,3 gew.-%ig) in 128 g 25 gew.-%igem wäßrigen Ammoniak bei 20°C in 2 h und anschließender Verseifung mit 50 g 50 gew.-%iger wäßriger Natronlauge für 20h bei 20°C, 3 h unter Stickstoffstrippung bei 95°C und anschließende Neutralisation auf pH 6 mit 44 g 50 gew.-%iger wäßriger Schwefelsäure, wurden gleichzeitig 28,5 g Blausäure (99,3 gew.-%ig) und 105 g Formaldehyd (30 gew.-%ig, wäßrig) getropft und noch 3 h bei 30°C nachgerührt. Die Abnahme an Blausäure entsprach 98 % der Theorie, bezogen auf Alanin.

Diese Lösung wurde daraufhin zu 103 g einer 50 gew.-%igen wäßrigen Natronlauge bei 30°C getropft und 4 h bei 30°C sowie weitere 6 h unter Strippung mit Stickstoff bei 95°C gerührt. Es entstanden 389 g einer gemäß Eisenbindevermögen 33,4 gew.-%igen wäßrigen Lösung von MGDA-Trinatriumsalz, entsprechend einer Ausbeute von 95 %, bezogen auf Alanin. Die Gesamtausbeute betrug damit 77 %, gegenüber 69 % bei vorheriger Isolierung von Alanin. Der Gehalt an NTA in der Lösung betrug 0,1 % Gew.

### Beispiel 2

### Herstellung von MGDA-Trinatriumsalz aus Alaninnitril-Rohgemisch

Zu 204 g 25 gew.-%igem wäßrigen Ammoniak wurden bei 0°C 27,2 g Blausäure (99,3 gew.-%ig) getropft. Zu der entstandenen Lösung wurden dann innerhalb von 20 min 44 g Acetaldehyd bei 0 bis 10°C getropft, nach weiteren 2 h bei 20°C betrug der Umsatz an HCN 98 %, anschließend wurde die Lösung 1 h bei 2 x 10⁴ Pa (200 mbar) mit Stickstoff gestrippt. Danach wurde mit 69 g 98 gew.-%iger Schwefelsäure ein pH-Wert von 2 eingestellt und innerhalb 30 min wurden gleichzeitig 55 g Blausäure (99,3 gew.-%ig) und 200 g Formaldehyd (30 gew.-%ig, wäßrig) zugetropft, es wurde noch weitere 8 h bei 50°C gerührt. Nach dem Erkalten fielen insgesamt 115 g, entsprechend 78 % der Theorie, Methylglycinnitril-N,N-diacetonitril als farbloser Niederschlag an. Durch Eintragen dieses Niederschlages in 471 g 20 gew.-%ige wäßrige Natronlauge bei 40°C, anschließendem 3 stündigem Rühren bei dieser Temperatur sowie weiteren 5 h bei 95°C (unter gleichzeitiger Strippung mit Stickstoff) erhielt man 553 g einer nach Eisenbindevermögen 35 gew.-%igen wäßrigen Lösung von MGDA-Trinatriumsalz, entsprechend einer Gesamtausbeute von 72 % der Theorie. Der NTA-Gehalt der Lösung betrug 0,07 Gew.-%.

### Beispiel 3

### Herstellung von MGDA-Trinatriumsalz aus Iminodiacetonitril-Rohgemisch (Weg a)

Zu 600 g 30 gew.-%igem wäßrigen Formaldehyd wurden 284 g 25 gew.-%iger wäßriger Ammoniak gegeben und anschließend der pH-Wert mit Schwefelsäure auf 6 eingestellt, dazu wurden bei 50°C 170 g Blausäure (99,1 gew.-%ig) und weitere Schwefelsäure zur Konstanthaltung des obigen pH-Wertes gegeben. Nach insgesamt 4 h wurde der pH-Wert durch Zugabe von Schwefelsäure auf 1,5 eingestellt und gleichzeitig wurden 82 g Blausäure (99,1 %ig) und 132 g Acetaldehyd zugegeben. Nach 4 h wurden 408 g Methylglycinnitril-N,N-diacetonitril kristallin erhalten, entsprechend 92 % der Theorie.

Durch Eintragen dieses Niederschlages in 1670 g 20 gew.-%ige wäßrige Natronlauge bei 40°C, anschließend 3 stündigem Rühren bei dieser Temperatur sowie weiteren 5 h bei 95°C (unter gleichzeitiger Strippung mit Stickstoff) erhielt man 1960 g einer nach Eisenbindevermögen 35 gew.%-igen wäßrigen Lösung von MGDA-Trinatriumsalz, entsprechend einer Gesamtausbeute von 85 % der Theorie. Der NTA-Gehalt der Lösung betrug 0,08 Gew.-%. Im Vergleich wird bei Zwischenisolierung von Iminodiacetonitril eine Gesamtausbeute von nur 70 % erhalten.

### Beispiel 4

### Herstellung von MGDA-Trinatriumsalz aus Iminodiessigsäure-Rohgemisch (Weg b)

95 g Iminodiacetonitril wurden in 420 g 50 gew.-%ige wäßrige Natronlauge bei 40°C eingetragen, nach 2 h wurde die Mischung für 5 h auf 95°C erhitzt. Die erhaltenen Iminodiessigsäure-Dinatriumsalz-Lösung wurde dann mit 49 g Schwefelsäure (98 gew.-%ig) auf pH 6 eingestellt und anschließend wurden bei 30°C gleichzeitig 28,5 g Blausäure (99,3 gew.-%ig) und 46 g Acetaldehyd zugetropft, danach wurde noch für 8 h bei 60°C nachgerührt. Die Abnahme an Blausäure entsprach 95 % der Theorie.

Diese Lösung wurde daraufhin zu 84 g einer 50 gew.-%igen wäßrigen Natronlauge bei 30°C getropft und 4 h bei 30°C sowie weitere 6 h unter Strippung mit Stickstoff bei 95°C gerührt. Es entstanden 661 g einer gemäß Eisenbindevermögen 36,5 gew.-%igen wäßrigen Lösung von MGDA-Trinatriumsalz, entsprechend einer Gesamtausbeute von 89 %, gegenüber 84 % bei vorheriger Isolierung von Iminodiessigsäure. Der Gehalt an NTA in der Lösung betrug 0,32 Gew.-%.

## Patentansprüche

1. Verfahren zur Herstellung von Glycin-N,N-diessigsäure-Derivaten der allgemeinen Formel I 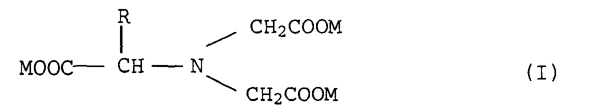 in der
R für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, Alkoxylat-Gruppierungen der Formel -(CH₂)ₖ-O-(A¹O)ₘ-(A²O)ₙ-Y, in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, Phenyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen oder C₁- bis C₄-Alkoxycarbonylgruppen tragen können, oder einen Rest der Formel 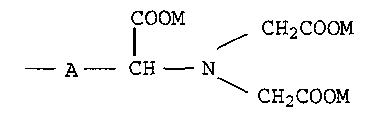 steht, wobei A eine C₁- bis C₁₂-Alkylen-Brücke oder eine chemische Bindung bezeichnet, und
M Wasserstoff, Alkalimetall, Erdalkalimetall, Ammonium oder substituiertes Ammonium in den entsprechenden stöchiometrischen Mengen bedeutet,
durch Umsetzung von
A) entsprechenden 2-substituierten Glycinen oder 2-substituierten Glycinnitrilen oder verdoppelten Glycinen der Formel 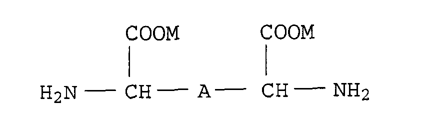 oder verdoppelten Glycinnitrilen der Formel 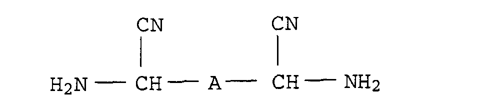 oder von Alaninaminonitril oder 5-Methylhydantoin als Vorstufen der als Ausgangsmaterial genannten Glycinderivate mit Formaldehyd und Cyanwasserstoff in wäßrigem Medium bei einem pH-Wert von 0 bis 8 oder
B)a) Iminodiacetonitril mit entsprechenden Monoaldehyden der Formel R-CHO oder Dialdehyden der Formel OHC-A-CHO und Cyanwasserstoff in wäßrigem Medium bei einem pH-Wert von 0 bis 5 oder
B)b) Iminodiessigsäure mit entsprechenden Monoaldehyden der Formel R-CHO oder Dialdehyden der Formel OHC-A-CHO und Cyanwasserstoff in wäßrigem Medium bei einem Ausgangs-pH-Wert von 1 bis 8
und gegebenenfalls anschließende Verseifung vorliegender Nitrilfunktionen, dadurch gekennzeichnet, daß man als Ausgangsmaterial aus der technischen Synthese von Glycinderivaten oder deren Vorstufen oder von Iminodiacetonitril oder Iminodiessigsäure stammendes nicht gereinigtes Rohmaterial oder bei solchen Synthesen anfallende Mutterlaugen einsetzt.

2. Verfahren zur Herstellung von Glycin-N,N-diessigsäure-Derivaten I nach Anspruch 1, bei denen R für C₁- bis C₂₀-Alkyl, C₂- bis C₂₀-Alkenyl oder einen Rest der Formel 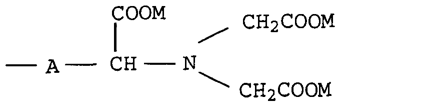 steht.

3. Verfahren zur Herstellung von α-Alanin-N,N-diessigsäure und ihren Alkalimetall-, Ammonium- und substituierten Ammoniumsalzen nach Anspruch 1.

## Claims

1. A process for preparing glycine-N,N-diacetic acid derivatives of the general formula I 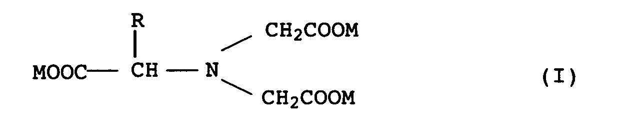 where
R is C₁-C₃₀-alkyl or C₂-C₃₀-alkenyl, each of which can additionally carry as substituents up to 5 hydroxyl groups, formyl groups, C₁-C₄-alkoxy groups, phenoxy groups or C₁-C₄-alkoxycarbonyl groups and can be interrupted by up to 5 nonadjacent oxygen atoms, or alkoxylate groups of the formula -(CH₂)ₖ-O-(A¹O)ₘ-(A²O)ₙ-Y, where A¹ and A² are, independently of one another, 1,2-alkylene groups having 2 to 4 carbon atoms, Y is hydrogen, C₁-C₁₂-alkyl, phenyl or C₁-C₄-alkoxycarbonyl, and k is 1, 2 or 3 and m and n are each from 0 to 50, it being necessary for the total of m + n to be at least 4, phenylalkyl groups having 1 to 20 carbon atoms in the alkyl, phenyl, a five- or six-membered unsaturated or saturated heterocyclic ring which has up to three hetero atoms from the group consisting of nitrogen, oxygen and sulfur and which can additionally be benzo-fused, it also being possible for all the phenyl nuclei and heterocyclic rings mentioned in the meanings for R additionally to carry as substituents up to three C₁-C₄-alkyl groups, hydroxyl groups, carboxyl groups, sulfo groups or C₁-C₄-alkoxycarbonyl groups, or a radical of the formula 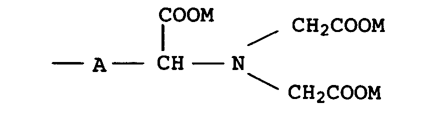 where A is a C₁-C₁₂-alkylene bridge or a chemical bond, and
M is hydrogen, alkali metal, alkaline earth metal, ammonium or substituted ammonium in the appropriate stoichiometric amounts,
by reacting
A) corresponding 2-substituted glycines or 2-substituted glycinonitriles or doubled glycines of the formula 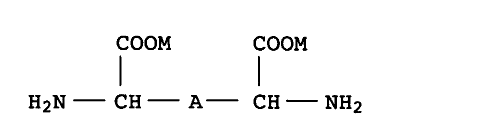 or doubled glycinonitriles of the formula 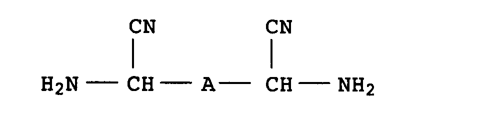 or alanine amino nitrile or 5-methylhydantoin as precursors of the glycine derivatives named as starting material with formaldehyde and hydrogen cyanide in aqueous medium at a pH of from 0 to 11 or
B) a) iminodiacetonitrile with appropriate monoaldehydes of the formula R-CHO or dialdehydes of the formula OHC-A-CHO and hydrogen cyanide in aqueous medium at a pH of from 0 to 5 or
B) b) iminodiacetic acid with appropriate monoaldehydes of the formula R-CHO or dialdehydes of the formula OHC-A-CHO and hydrogen cyanide in aqueous medium at a starting pH of from 1 to 8
and, where appropriate, subsequent hydrolysis of nitrile functionalities which are present, wherein the starting material used comprises unpurified raw material derived from the industrial synthesis of glycine derivatives or their precursors or of iminodiacetonitrile or iminodiacetic acid, or mother liquors produced in such syntheses.

2. A process for preparing glycine-N,N-diacetic acid derivatives I as claimed in claim 1, where R is C₁-C₂₀-alkyl, C₂-C₂₀-alkenyl or a radical of the formula

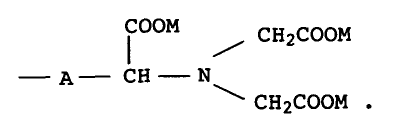

3. A process for preparing α-alanine-N,N-diacetic acid and its alkali metal, ammonium and substituted ammonium salts as claimed in claim 1.

## Revendications

1. Procédé de préparation de dérivés de glycine-N,N-diacétique de formule générale I 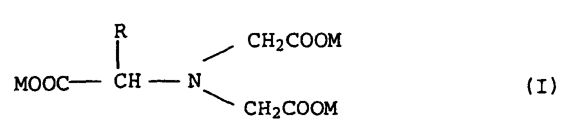 dans laquelle
R représente des groupements alkyle en C₁-C₃₀ ou alcényle en C₂-C₃₀, pouvant en outre porter en tant que substituants jusqu'à 5 groupements hydroxyle, formyle, alcoxy en C₁-C₄, phénoxy ou alcoxycarbonyle en C₁-C₄ et pouvant être interrompus par jusqu'à 5 atomes d'oxygène non voisins, des groupements alcoxylate de formule -(CH₂)ₖ-O-(A¹O)ₘ-(A²O)ₙ-Y, dans laquelle A¹ et A² représentent indépendamment les uns des autres, des groupements 1,2-alkylène ayant 2 à 4 atomes de carbone, Y représente un atome d'hydrogène, des groupements alkyle en C₁-C₁₂, phényle ou alcoxycarbonyle en C₁-C₄, et k est mis pour le nombre 1, 2 ou 3 tandis que m et n sont mis chacun pour des nombres de 0 à 50, où la somme m + n vaut au moins 4, des groupements phénylalkyle comportant 1 à 20 atomes de carbone dans la fraction alkyle, phényle, un cycle hétérocyclique saturé ou insaturé à 5 ou 6 maillons ayant jusqu'à trois hétéroatomes choisis dans le groupe de l'azote, de l'oxygène et du soufre, pouvant en outre être benzocondensé, où tous les noyaux phényle et cycles hétérocycliques mentionnés pour les significations de R peuvent en outre porter en tant que substituants jusqu'à trois groupements alkyle en C₁-C₄, hydroxyle, carboxyle, sulfo ou alcoxycarbonyle en C₁-C₄, ou un reste de formule 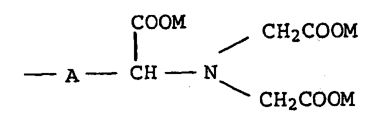 où A représente un pont alkylène en C₁-C₁₂ ou une liaison chimique, et
M représente un atome d'hydrogène, un atome de métal alcalin, un atome de métal alcalino-terreux, un ammonium ou un ammonium substitué dans les quantités stoechiométriques correspondantes,
par réaction de
A) glycines 2-substituées ou de glycine-nitriles 2-substituées ou de glycines doubles correspondantes de formule 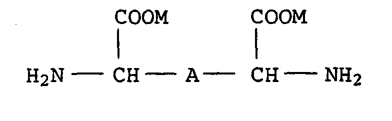 ou de glycine-nitriles doubles de formule 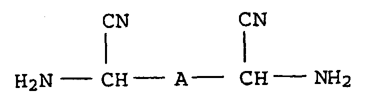 ou d'alanine-aminonitrile ou de 5-méthylhydanthoïne en tant que précurseurs des dérivés de glycine mentionnés comme matériaux de départ, avec du formaldéhyde et de l'acide cyanhydrique en milieu aqueux à une valeur de pH de 0 à 8 ou
B) a) d'iminodiacétonitrile avec les monoaldéhydes de formule R-CHO ou les dialdéhydes de formule OHC-A-CHO correspondants et de l'acide cyanhydrique en milieu aqueux à une valeur de pH de 0 à 5 ou
B) b) d'acide iminodiacétique avec les monoaldéhydes de formule R-CHO ou les dialdéhydes de formule OHC-A-CHO correspondants et de l'acide cyanhydrique en milieu aqueux à une valeur de pH de départ de 1 à 8
éventuellement suivie d'une saponification des fonctions nitrile présentes, caractérisé en ce que l'on utilise en tant que matières de départ des matières brutes non purifiées issues de la synthèse technique de dérivés de glycine ou de leurs précurseurs ou d'iminodiacétonitrile ou d'acide iminodiacétique, ou bien des solutions mères produites lors de telles synthèses.

2. Procédé de préparation de dérivés de glycine-N,N-diacétique I selon la revendication 1, pour lesquels R représente des groupements alkyle en C₁-C₂₀, alcényle en C₂-C₂₀ ou un reste de formule

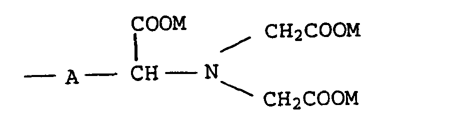

3. Procédé de préparation d'acide α-alanine-N,N-diacétique et de ses sels de métal alcalin, d'ammonium et de ses sels d'ammonium substitués selon la revendication 1.
